# EUROPEAN PATENT SPECIFICATION

(11) **EP 2 440 242 B1**
(45) Date of publication and mention of the grant of the patent: **25.06.2014**
(21) Application number: 10721036.1
(22) Date of filing: 08.06.2010
(51) Int. Cl.: A61K 39/00

(54) **ADJUVANT COMPOSITIONS COMPRISING A NON-IONIC ISOTONICITY AGENT**
ADJUVANSZUSAMMENSETZUNGEN ENTHALTEND EIN NICHTIONISCH ISOTONIZITÄTSMITTEL
COMPOSITIONS D'ADJUVANT COMPRENANT UN AGENT ISOTONIQUE NON IONIQUE

(30) Priority: 10.06.2009 GB 0910046
(43) Date of publication of application: 18.04.2012
(73) Proprietor: GlaxoSmithKline Biologicals S.A., 1330 Rixensart (BE)
(72) Inventor: HENDERICKX, Veronique, B-1330 Rixensart (BE); LEMOINE, Dominique Ingrid, B-1330 Rixensart (BE)
(74) Representative: Robertson, James Stuart
(86) International application number: PCT/EP2010/058017
(87) International publication number: WO 2010/142685

(56) References cited:
- WO-A-2008/142133
- WO-A1-96/33739
- WO-A2-99/56776
- US-B1- 6 869 607

## Description

### Field of the Invention

The present invention relates to aqueous adjuvant compositions comprising a non-ionic isotonicity agent and having low concentrations of salt, in particular having sodium chloride concentrations at or below 100mM. The present invention also relates to immunogenic compositions comprising an antigen or antigen preparation and said aqueous adjuvant compositions.

### Background of the Invention

Adjuvants are sometimes used to improve the immune response raised to any given antigen. However the inclusion of adjuvants into a vaccine or immunogenic composition increases the complexity of preparation of the components as well as the complexity of distribution and formulation of the vaccine composition. The preparation of each of the adjuvant components as well as the antigenic component must be considered by formulators. In particular, the compatibility of the antigenic component with the adjuvant component should be considered. This is particularly the case where lyophilised antigens or antigenic preparations are intended to be reconstituted with an adjuvant preparation. In such a circumstance, it is important that the buffer of the adjuvant preparation is suitable for the antigen or antigenic preparation and that immunogenicity or solubility of the antigen is not affected by the adjuvant. US6,868,607 discloses vaccines comprising peptides, a polycation adjuvant and one or more water soluble or water emulsifiable substances which is capable of making the vaccine isotonic.

### Summary of the Invention

The present inventors have found that some antigens are particularly sensitive to a phenomenon known as "salting out" which may be defined as the precipitation of a protein from its solution by saturation with a salt such as sodium chloride. The present inventors have found that sensitive antigens may aggregate and precipitate at a concentration of sodium chloride as low as 150mM.

Therefore the present invention provides an aqueous isotonic adjuvant composition comprising a Toll-like receptor (TLR) 4 agonist, and a saponin in a liposomal formulation and a non-ionic isotonicity agent, wherein the concentration of sodium chloride in said composition is less than 100mM.

The present invention further provides an aqueous isotonic adjuvant composition comprising a TLR-4 agonist, and a saponin in a liposomal formulation and a non-ionic isotonicity agent wherein the ionic strength in said composition is less than 100mM.

In addition, the present invention provides an aqueous isotortic adjuvant composition which can be used for a broader range of protein antigens including those that are susceptible to "salting out" as well that are not.

The present invention also provides an immunogenic composition comprising an antigen or antigenic preparation and an aqueous adjuvant composition comprising a TLR-4 agonist, and a saponin in a liposomal formulation and a non-ionic isotonic agent, wherein the concentration of sodium chloride in said adjuvant composition is less than 100mM and processes for making said immunogenic compositions.

### Brief description of the drawings.

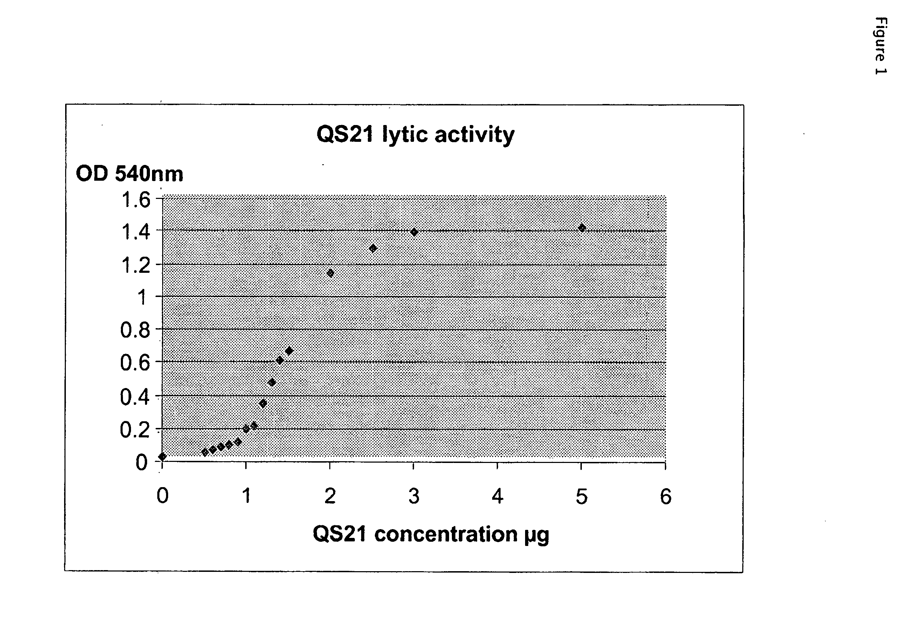

**Figure 1****.** QS21 lytic activity curve.
**Figure 2****.** Percentage of each 3D-MPL congener in the different ASA formulations.
**Figure 3****.** Freeze-drying cycle used for lyophilisation of PRAME/CpG.
**Figure 4****.** A pictorial comparison between PRAME and NYESO-1 reconstituted in ASA (150mM NaCl) and ASA (sorbitol).
**Figure 5****.** Humoral response of mice immunised with PRAME/CpG formulated with differing adjuvant compositions in Experiment 1.
**Figure 6****.** Tumor protection in mice immunised with PRAME/CpG formulated with differing adjuvant compositions in Experiment 1.
**Figure 7****.** Humoral response of mice immunised with PRAME/CpG formulated with ASA (150mM NaCl), ASA (sorbitol) or liquid formulation ASA (70mM NaCl) in Experiment 2.
**Figure 8****.** CD4+ response of mice immunised with PRAME/CpG formulated with ASA (150mM NaCl), ASA (sorbitol) or liquid formulation ASA in Experiment 2.
**Figure 9****.** Tumor protection in mice immunised with PRAME/CpG formulated with ASA (150mM NaCl), ASA (sorbitol) or liquid formulation ASA in Experiment 2.

### Detailed Description of the Invention

The present invention describes the replacement or partial replacement of an isotonicity agent which is a salt such as sodium chloride in an aqueous adjuvant composition with a non-ionic isotonicity agent.

It is well known that for parenteral administration solutions should be physiologically isotonic (*i.e*. have a pharmaceutically acceptable osmolality) to avoid cell distortion or lysis. An "isotonicity agent" is a compound that is physiologically tolerated and imparts a suitable tonicity to a formulation (e.g. immunogenic compositions of the invention) to prevent the net flow of water across cell membranes that are in contact with the formulation.

Aqueous adjuvant compositions are known which contain 100 mM sodium chloride or more, for example adjuvant system A (ASA) in WO 2005/112991 and WO2008/142133 or the liposomal adjuvants disclosed in WO2007/068907. As set out in WO2008/142133, such adjuvant compositions may be used as diluent to reconstitute lyophilised compositions comprising antigens or antigenic preparations prior to vaccination. It is important that such reconstituted compositions are isotonic, *i.e.* contain a salt concentration substantially the same as that found in the cells of the body and the blood such that no cell shrinkage or expansion is caused on injection. Generally, sodium chloride is used as an isotonicity agent. The present inventors have found that certain antigens are particularly sensitive to "salting out", a process whereby proteins in solution aggregate or coagulate when in solutions containing high concentrations of salt.

The concentrations of salt at which protein antigens aggregate varies from protein to protein. The present inventors have identified a group of antigens which will aggregate at relatively low concentrations of salt, for example at about 100mM or less of sodium chloride. This means that certain known adjuvant compositions are not suitable for reconstitution of or use with compositions comprising these antigens as aggregation occurs.

The present invention provides aqueous adjuvant compositions which may be used with such antigens, *i.e.* those antigens that aggregate at salt concentrations of below 100mM sodium chloride. Aqueous adjuvant compositions of the invention comprise a TLR-4 agonist, and a saponin in a liposomal formulation and a non-ionic isotonicity agent wherein the concentration of sodium chloride in the adjuvant composition is below about 100mM, for example below 90mM, 80mM, 70mM, 60mM, 50mM, 40mM, 30mM, 20 mM or 15 mM. In a particular embodiment the concentration of sodium chloride in the adjuvant composition is below 10mM or is at or below 5mM. In a further specific embodiment, the adjuvant composition is essentially free of sodium chloride. By essentially free is meant that the concentration of sodium chloride is at or very near to zero mM (*i.e.* 1mM, 2mM, or 3mM).

The skilled person can readily test for the concentration of both sodium (Na⁺) and chloride (Cl⁻) ions using known techniques and kits. For example, sodium can be determined using a kit such as the Sodium Enzymatic Assay Kit (Catalog Number: BQ011EAEL) from Biosupply. Chloride can be determined using a kit such as Chloride Enzymatic Assay Kit (Catalog Number: BQ006EAEL) from Biosupply.

The present invention further provides an aqueous isotonic adjuvant composition comprising a TLR-4 agonist, and a saponin in a liposomal formulation and a non-ionic isotonicity agent wherein the ionic strength is less than 100mM, for example below 90mM, 80mM, 70mM, 60mM, 50mM, 40mM, 30mM, 20 mM or 15 mM. In a particular embodiment the ionic strength in the adjuvant composition is below 10mM or is at or below 5mM. In a further specific embodiment, the adjuvant composition has an ionic strength that is at or very near to zero mM.

The ionic strength of an adjuvant or immunogenic composition of the invention can be measured using techniques known the skilled person, for example using a conductivity meter.

A suitable non-ionic isotonicity agent for use in an aqueous adjuvant composition of the invention which is to be combined with an antigenic composition will need to be suitable for use in humans, as well as being compatible with the antigens within the antigenic composition and further compatible with other components of the adjuvant composition.

In particular, the aqueous adjuvant composition must be such that the antigens within the antigenic composition, when combined with the adjuvant composition, are able to both remain in solution and retain their immunogenicity.

In one embodiment of the present invention, suitable non-ionic isotonicity agents are polyols, sugars (in particular sucrose, fructose, dextrose or glucose) or amino acids such as glycine. In one embodiment the polyol is a sugar alcohol especially a C3-6 sugar alcohol. Exemplary sugar alcohols include glycerol, erythritol, threitol, arabitol, xylitol, ribitol, sorbitol, mannitol, dulcitol and iditol. In a specific example of this embodiment, a suitable non-ionic isotonicity agent is sorbitol. In a particular embodiment of the invention the non-ionic isotonicity agent in the compositions of the invention is sucrose and/or sorbitol.

In one embodiment, a suitable concentration of polyol within the aqueous adjuvant composition is between about 3 and about 15% (w/v), in particular between about 3 and about 10% (w/v) for example between about 3 and about 7 % (w/v), for example between about 4 and about 6% (w/v). In a specific example of this embodiment, the polyol is sorbitol.

The aqueous adjuvant composition comprises a Toll-like receptor agonist (TLR) 4 agonist, and a saponin in a liposomal formulation. By this it is meant that the saponin and TLR-4 agonist are formulated with liposomes.

The term "liposomes" generally refers to uni- or multilamellar (particularly 2, 3, 4, 5, 6, 7, 8, 9, or 10 lamellar depending on the number of lipid membranes formed) lipid structures enclosing an aqueous interior. Liposomes and liposome formulations are well known in the art. Lipids, which are capable of forming liposomes include all substances having fatty or fat-like properties. Lipids which can make up the lipids in the liposomes can be selected from the group comprising of glycerides, glycerophospholipides, glycerophosphinolipids, glycerophosphonolipids, sulfolipids, sphingolipids, phospholipids, isoprenolides, steroids, stearines, sterols, archeolipids, synthetic cationic lipids and carbohydrate containing lipids.

In one embodiment the liposomes comprise a phospholipid. Suitable phospholipids include (but are not limited to): phosphocholine (PC) which is an intermediate in the synthesis of phosphatidylcholine; natural phospholipid derivates: egg phosphocholine, egg phosphocholine, soy phosphocholine, hydrogenated soy phosphocholine, sphingomyelin as natural phospholipids; and synthetic phospholipid derivates: phosphocholine (didecanoyl-L-α-phosphatidylcholine [DDPC], dilauroylphosphatidylcholine [DLPC], dimyristoylphosphatidylcholine [DMPC], dipalmitoyl phosphatidylcholine [DPPC], Distearoyl phosphatidylcholine [DSPC], Dioleoyl phosphatidylcholine [DOPC], 1-palmitoyl, 2-oleoylphosphatidylcholine [POPC], Dielaidoyl phosphatidylcholine [DEPC]), phosphoglycerol (1,2-Dimyristoyl-sn-glycero-3-phosphoglycerol [DMPG], 1,2-dipalmitoyl-sn-glycero-3-phosphoglycerol [DPPG], 1,2-distearoyl-sn-glycero-3-phosphoglycerol [DSPG], 1-palmitoyl-2-oleoyl-sn-glycero-3-phosphoglycerol [POPG]), phosphatidic acid (1,2-dimyristoyl-sn-glycero-3-phosphatidic acid [DMPA], dipalmitoyl phosphatidic acid [DPPA], distearoyl-phosphatidic acid [DSPA]), phosphoethanolamine (1,2-dimyristoyl-sn-glycero-3-phosphoethanolamine [DMPE], 1,2-Dipalmitoyl-sn-glycero-3-phosphoethanolamine [DPPE], 1,2-distearoyl-sn-glycero-3-phosphoethanolamine DSPE 1,2-Dioleoyl-sn-Glycero-3-Phosphoethanolamine [DOPE]), phoshoserine, polyethylene glycol [PEG] phospholipid (mPEG-phospholipid, polyglycerin-phospholipid, funcitionilized-phospholipid, terminal activated-phosholipid). In one embodiment the liposomes comprise 1-palmitoyl-2-oleoyl-glycero-3-phosphoethanolamine. In one embodiment highly purified phosphatidylcholine is used and can be selected fom the group comprising Phosphatidylcholine (EGG), Phosphatidylcholine Hydrogenated (EGG) Phosphatidylcholine (SOY) Phosphatidylcholine Hydrogenated (SOY). In a further embodiment the liposomes comprise phosphatidylethanolamine [POPE] or a derivative thereof.

Liposome size may vary from 30 nm to several µm depending on the phospholipid composition and the method used for their preparation. In particular embodiments of the invention, the liposome size will be in the range of 50 nm to 500 nm and in further embodiments 50 nm to 200 nm. Dynamic laser light scattering is a method used to measure the size of liposomes well known to those skilled in the art.

The liposomes suitably contain a neutral lipid, for example phosphatidylcholine, which is suitably non-crystalline at room temperature, for example eggyolk phosphatidylcholine, dioleoyl phosphatidylcholine (DOPC) or dilauryl phosphatidylcholine. In a particular embodiment, the liposomes of the present invention contain DOPC. The liposomes may also contain a charged lipid which increases the stability of the lipsome-saponin structure for liposomes composed of saturated lipids. In these cases the amount of charged lipid is suitably 1 to 20% w/w, preferably 5 to 10%. The ratio of sterol to phospholipid is 1 to 50% (mol/mol), suitably 20 to 25%.

A particularly suitable saponin for use in the present invention is Quil A and its derivatives. Quil A is a saponin preparation isolated from the South American tree *Quillaja Saponaria Molina* and was first described by Dalsgaard et al. in 1974 ("Saponin adjuvants", Archiv. für die gesamte Virusforschung, Vol. 44, Springer Verlag, Berlin, p243-254) to have adjuvant activity. Purified fragments of Quil A have been isolated by HPLC which retain adjuvant activity without the toxicity associated with Quil A (EP 0 362 278), for example QS7 and QS21 (also known as QA7 and QA21). QS-21 is a natural saponin derived from the bark of *Quillaja saponaria* Molina, which induces CD8+ cytotoxic T cells (CTLs), Th1 cells and a predominant IgG2a antibody response. QS21 is a preferred saponin in the context of the present invention.

In a suitable form of the present invention, the saponin adjuvant within the immunogenic composition is a derivative of *saponaria molina* quil A, preferably an immunologically active fraction of Quil A, such as QS-17 or QS-21, suitably QS-21.

In a specific embodiment, QS21 is provided in its less reactogenic composition where it is quenched with an exogenous sterol, such as cholesterol for example. Several particular forms of less reactogenic compositions wherein QS21 is quenched with an exogenous cholesterol exist. The saponin/sterol is in a liposomal formulation structure (WO 96/33739, Example 1).

Suitable sterols include β-sitosterol, stigmasterol, ergosterol, ergocalciferol and cholesterol. In one particular embodiment, the adjuvant composition comprises cholesterol as sterol. These sterols are well known in the art, for example cholesterol is disclosed in the Merck Index, 11th Edn., page 341, as a naturally occurring sterol found in animal fat.

Where the active saponin fraction is QS21, the ratio of QS21 : sterol will typically be in the order of 1:100 to 1:1 (w/w), suitably between 1:10 to 1:1 (w/w), and preferably 1:5 to 1:1 (w/w). Suitably excess sterol is present, the ratio of OS21:sterol being at least 1:2 (w/w). In one embodiment, the ratio of QS21:sterol is 1:5 (w/w). The sterol is suitably cholesterol.

The aqueous adjuvant composition comprises a Toll-like receptor 4 (TLR-4) agonist. By "TLR agonist" it is meant a component which is capable of causing a signalling response through a TLR signalling pathway, either as a direct ligand or indirectly through generation of endogenous or exogenous ligand (Sabroe et al, JI 2003 p1630-5). A TLR4 agonist is capable of causing a signally response through a TLR-4 signalling pathway. A suitable example of a TLR-4 agonist is a lipopolysaccharide, suitably a non-toxic derivative of lipid A, particularly monophosphoryl lipid A or more particularly 3-Deacylated monophoshoryl lipid A (3D - MPL).

3D-MPL is sold under the name MPL by GlaxoSmithKline Biologicals N.A. and is referred throughout the document as MPL or 3D-MPL. see, for example, US Patent Nos. 4,436,727; 4,877,611; 4,866,034 and 4,912,094. 3D-MPL primarily promotes CD4+ T cell responses with an IFN-g (Th1) phenotype. 3D-MPL can be produced according to the methods disclosed in GB 2 220 211 A. Chemically it is a mixture of 3-deacylated monophosphoryl lipid A with 4, 5 or 6 acylated chains. In the compositions of the present invention small particle 3D-MPL may be used to prepare the aqueous adjuvant composition. Small particle 3D-MPL has a particle size such that it may be sterile-filtered through a 0.22µm filter. Such preparations are described in WO 94/21292. Preferably, powdered 3D-MPL is used to prepare the aqueous adjuvant compositions of the present invention.

Other TLR-4 agonists which can be used are alkyl Glucosaminide phosphates (AGPs) such as those disclosed in WO98/50399 or US patent No. 6,303, 347 (processes for preparation of AGPs are also disclosed), suitably RC527 or RC529 or pharmaceutically acceptable salts of AGPs as disclosed in US Patent No. 6,764,840.

Other suitable TLR-4 agonists are as described in WO2003/011223 and in WO 2003/099195, such as compound I, compound II and compound III disclosed on pages 4-5 of WO2003/011223 or on pages 3 to 4 of WO2003/099195 and in particular those compounds disclosed in WO2003/011223 as ER803022, ER803058, ER803732, ER804053, ER804057m ER804058, ER804059, ER804442, ER804680 and ER804764. For example, one suitable TLR-4 agonist is ER804057.

Aqueous adjuvant compositions of the invention comprise both a saponin and a TLR-4 agonist. In a particular embodiment, the aqueous adjuvant composition comprises QS21 and 3D-MPL.

A TLR-4 agonist such as a lipopolysaccharide, such as 3D-MPL, can be used at amounts between 1 and 100µg per human dose of the adjuvant composition. 3D-MPL may be used at a level of about 50µg, for example between 40 to 60 µg, suitably between 45 to 55 µg or between 49 to 51 µg or 50µg. In a further embodiment, the human dose of the adjuvant composition comprises 3D-MPL at a level of about 25µg, for example between 20 to 30µg, suitably between 21 to 29µg or between 22 to 28µg or between 28 to 27µg or between 24 to 26µg, or 25µg.

A saponin, such as QS21, can be used at amounts between 1 and 100µg per human dose of the adjuvant composition. QS21 may be used at a level of about 50µg, for example between 40 to 60 µg, suitably between 45 to 55 µg or between 49 and 51 µg or 50µg. In a further embodiment, the human dose of the adjuvant composition comprises QS21 at a level of about 25µg, for example between 20 to 30µg, suitably between 21 to 29µg or between 22 to 28µg or between 28 and 27µg or between 24 and 26µg, or 25µg.

Both TLR4 agonist and saponin are present in the aqueous adjuvant composition - the weight ratio of TLR4 agonist to saponin is suitably between 1:5 to 5:1, suitably 1:1. For example, where 3D-MPL is present at an amount of 50µg or 25µg, then suitably QS21 may also be present at an amount of 50µg or 25µg, respectively, per human dose of the aqueous adjuvant composition.

When the adjuvant is to be combined with a liquid form of an antigenic composition, the adjuvant composition will be in a human dose suitable volume which is approximately half of the intended final volume of the human dose. For example a 500 µl volume of adjuvant for an intended final human dose of 1 ml, or a 250 µl volume for an intended final human dose of 0.5 ml. The adjuvant composition is diluted when combined with the antigen composition to provide the final human dose of vaccine. The final volume of such dose will of course vary dependent on the intial volume of the adjuvant composition and the volume of antigen composition added to the adjuvant composition. In an alternative embodiment, the aqueous adjuvant is used to reconstitute a lyophilised antigen composition. In this embodiment, the human dose suitable volume of the adjuvant composition is approximately equal to the final volume of the human dose. The liquid adjuvant composition is added to the vial containing the lyophilised antigen composition and used to reconstitute the lyophilised antigen composition.

The present invention therefore provides a process for preparing an immunogenic composition comprising the steps of reconstituting a lyophilised composition comprising at least one antigen or antigenic preparation as described herein with an aqueous adjuvant composition as defined herein.

In a further embodiment of the invention, there is provided a kit comprising (i) a lyophilised composition comprising an antigen or antigenic preparation and (ii) an aqueous adjuvant composition as described herein.

In a particular embodiment of the invention, there is provided a kit comprising (i) a lyophilised composition comprising an antigen or antigenic preparation as described herein and (ii) an aqueous adjuvant composition as described herein.

In one embodiment, the lyophilised composition further comprises a TLR-9 agonist, for example as set out in WO 2008/142133.

In an alternative embodiment, there is a provided a kit wherein the CpG is not co-lyophilsed with the antigen. The CpG may be either mixed with the aqueous adjuvant composition, or be in a separate vial in aqueous or lyophilised form. Accordingly, in an alternative embodiment, there is provided a kit comprising (i) a lyophilised compositon comprising an antigen as described herein; (ii) an aqueous adjuvant composition; and (iii) a TLR9 agonist (for example an immunostimulatory CpG oligonucleotide).

The TLR-9 agonist for use in kits of the invention is an immunostimulatory oligonucleotide, in particular an oligonucleotide containing an unmethylated CpG motif. Such oligonucleotides are well known and are described, for example, in WO 96/02555, WO 99/33488 and US 5,865, 462. Suitable TLR9 agonists for use in the immunogenic compositions described herein are CpG containing oligonucleotides, optionally containing two or more dinucleotide CpG motifs separated by at least three, suitably at least six or more nucleotides. A CpG motif is a cytosine nucleotide followed by a Guanine nucleotide.

In one embodiment the internucleotide bond in the oligonucleotide is phosphorodithioate, or possibly a phosphorothioate bond, although phosphodiester and other internucleotide bonds could also be used, including oligonucleotides with mixed internucleotide linkages. Methods for producing phosphorothioate oligonucleotides or phosphorodithioate are described in US5,666,153, US5,278,302 and WO95/26204. Oligonucleotide comprising different internucleotide linkages are contemplated, e.g. mixed phosphorothioate phophodiesters. Other internucleotide bonds which stabilise the oligonucleotide may be used.

Examples of CpG oligonucleotides suitable for inclusion in the immunogenic compositions described herein have the following sequences. In one embodiment, these sequences contain phosphorothioate modified internucleotide linkages.

OLIGO 1(SEQ ID NO:1): TCC ATG ACG TTC CTG ACG TT (CpG 1826)
OLIGO 2 (SEQ ID NO:2): TCT CCC AGC GTG CGC CAT (CpG 1758)
OLIGO 3(SEQ ID NO:3): ACC GAT GAC GTC GCC GGT GAC GGC ACC ACG
OLIGO 4 (SEQ ID NO:4): TCG TCG TTT TGT CGT TTT GTC GTT (CpG 2006)
OLIGO 5 (SEQ ID NO:5): TCC ATG ACG TTC CTG ATG CT (CpG 1668)

Alternative CpG oligonucleotides may comprise the sequences above in that they have inconsequential deletions or additions thereto.

The present invention further provides an immunogenic composition comprising an antigen or antigenic preparation and an aqueous adjuvant composition as described herein wherein said immunogenic composition has a concentration of sodium chloride below about 100mM, for example below 90mM, 80mM, 70mM, 60mM, 50mM, 40mM, 30mM, 20 mM or 15 mM. In a particular embodiment the concentration of sodium chloride in the immunogenic composition is below 10mM or is at or below about 5mM. In a further specific embodiment, the immunogenic composition is essentially free of sodium chloride. By essentially free is meant that the concentration of sodium chloride is at or very near to zero mM.

The present invention further provides an immunogenic composition comprising an antigen or antigenic preparation and an aqueous adjuvant composition as described herein wherein the ionic strength of the immunogenic composition is less than 100mM, for example below 90mM, 80mM, 70mM, 60mM, 50mM, 40mM, 30mM, 20 mM or 15 mM. In a particular embodiment the ionic strength in the immunogenic composition is below 10mM or is at or below 5mM. In a further specific embodiment, the immunogenic composition has an ionic strength that is at or very near to zero mM.

It will be apparent that, if the immunogenic composition has been prepared using a lyophilised antigenic composition comprising a TLR-9 agonist, then the immunogenic composition will also comprise a TLR-9 agonist. Therefore in one embodiment is provided an immunogenic composition comprising an antigen or antigenic preparation, a TLR9 agonist, and a TLR-4 agonist and a saponin in a liposomal formulation, wherein the immunogenic composition has a salt concentration below about 100mM, for example below 90mM, 80mM, 70mM, 60mM, 50mM, 40mM, 30mM, 20 mM or 15 mM. In a specific example of this embodiment the concentration of sodium chloride in the immunogenic composition is below 10mM or is at or below about 5mM.

In a further embodiment, there is provided an immunogenic composition comprising an antigen or antigenic preparation, a TLR9 agonist, and a TLR-4 agonist and a saponin in a liposomal formulation wherein the ionic strength is less than 100mM, for example below 90mM, 80mM, 70mM, 60mM, 50mM, 40mM, 30mM, 20 mM or 15 mM. In a particular embodiment the ionic strength in the immunogenic composition is below 10mM or is at or below 5mM. In a further specific embodiment, the immunogenic composition has an ionic strength that is at or very near to zero mM.

In a further embodiment is provided an immunogenic composition comprising an antigen or antigenic preparation, a TLR9 agonist and a 3D-MPL and QS21 in a liposomal formulation, wherein the immunogenic composition has a salt concentration below about 100mM, for example below 90mM, 80mM, 70mM, 60mM, 50mM, 40mM, 30mM, 20 mM or 15 mM. In a specific example of this embodiment the concentration of sodium chloride in the immunogenic composition is below 10mM or is at or below about 5mM.

In a further embodiment is provided an immunogenic composition comprising an antigen or antigenic preparation, a TLR9 agonist and a 3D-MPL and QS21 in a liposomal formulation, wherein the ionic strength is less than 100mM, for example below 90mM, 80mM, 70mM, 60mM, 50mM, 40mM, 30mM, 20 mM or 15 mM. In a particular embodiment the ionic strength in the immunogenic composition is below 10mM or is at or below 5mM. In a further specific embodiment, the immunogenic composition has an ionic strength that is at or very near to zero mM.

In a further embodiment is provided an immunogenic composition comprising an antigen or antigenic preparation and 3D-MPL and QS21 in a liposomal formulation, and a CpG oligonucleotide, wherein the immunogenic composition has a salt concentration below about 50mM, for example below about 40mM, 30mM, 20 mM or 15 mM. In a specific example of this embodiment the concentration of sodium chloride in the immunogenic composition is below 10mM or is at or below 5mM.

In one embodiment, the antigen or antigenic preparation used in the immunogenic compositions of the invention is any antigen which precipitates, coagulates or aggregates after being mixed and/or dissolved with a solution comprising a concentration of sodium chloride greater than 50mM, 60mM, 70mM, 80mM, 90mM or 100mM.

In one embodiment, the antigen or antigenic preparation used in the immunogenic compositions of the invention is any antigen which precipitates, coagulates or aggregates after being mixed and/or dissolved a solution wherein the ionic strength is less than 100mM, for example below 90mM, 80mM, 70mM, 60mM, 50mM, 40mM, 30mM, 20mM, 15 mM or 10mM. In a particular embodiment the antigens of the invention precipitate, coagulate or aggregate in solutions with an ionic strength at or below 5mM.

The person skilled in the art can determine whether an antigen meets this definition by mixing the antigen in such a solution. An antigen which does not meet this definition will still be in solution, *i.e.* the liquid will still be clear with no precipitation, 24 hours after being dissolved. An antigen which precipitates, coagulates or aggregates after being mixed and/or dissolved in a solution, can be seen by visual inspection to be precipitating by the cloudiness of the solution. In addition, aggregation that is not detectable visually may be observed using methods known to the skilled person which include, but are not limited to, SEC-HPLC.

In a further embodiment the antigen or antigenic preparation is derived from HIV, *Neisseria meningitidis,* or is a tumour associated antigen. In a particular embodiment the tumour associated antigen is selected from either PRAME or NYESO-1 or a fragment or derivative thereof.

PRAME (also known as DAGE) is an antigen that may be used as the tumour associated antigen of the present invention. The antigen and its preparation are described in US patent No. 5, 830, 753. PRAME is found in the Annotated Human Gene Database H-Inv DB under the accession numbers: U65011.1, BC022008.1, AK129783.1, BC014974.2, CR608334.1, AF025440.1, CR591755.1, BC039731.1, CR623010.1, CR611321.1, CR618501.1, CR604772.1, CR456549.1, and CR620272.1.

Fusion proteins that comprise the PRAME antigen may also be used. PRAME or a fragment or derivative thereof may be employed, optionally in the form of a fusion protein with a heterologous fusion partner. In particular, PRAME antigen may suitably be employed in the form of a fusion protein with *Haemophilus influenzae B* protein D or a portion thereof or derivative thereof. The portion of protein D that may be employed suitably does not include the secretion sequence or signal sequence. Suitably the fusion partner protein comprises amino acids Met-Asp-Pro at or within the N-terminus of the fusion protein sequence and in which the fusion partner protein does not include the secretion sequence or the signal sequence of protein D. For example the fusion partner protein may comprise or consist of approximately or exactly amino acids 17 to 127, 18 to 127, 19 to 127 or 20 to 127 of protein D. Suitable PRAME antigens based on fusions proteins with protein D are described in WO2008/087102.

NY-ESO-1 is another antigen that may be used as the tumour associated antigen of the present invention. NY-ESO-1 or a fragment or derivative thereof may be employed, optionally in the form of a fusion protein with a heterologous fusion partner. NY-ESO-1 is described in US5804381. The protein NY-ESO-1 is approximately 180 amino acids in length and can be described as being composed of three regions: (a) an N-terminal region being about amino acids 1-70 (b) a central region being about amino acids 71-134 and (c) a C terminal region being about amino acids 135-180. NY-ESO-1 may be employed as a fusion protein for example as a fusion with LAGE-1 which is a further CT antigen, or a fragment thereof, see WO2008/089074. Where fragments of NY-ESO-1 are employed these suitably include one or more MHC Class 1 or Class 2 epitopes e.g. those known as A31, DR1, DR2, DR4, DR7, DP4, B35, B51, Cw3, Cw6 and A2 (see WO2008/089074).
Although not sensitive to NaCl as such, a further antigen that may be employed in accordance with the present invention is a MAGE antigen or a fragment or derivative thereof, *e.g.* of the MAGE-3 family such as MAGE-A3. MAGE-3 antigens have, for example, been described as suitable to be formulated in combination with NY-ESO-1 - see WO2005/105139.

MAGE antigens such as MAGE-A3 may be used as such or in the form of a derivative e.g. a chemically modified derivative and/or in the form of a fusion protein with a heterologous fusion partner. For example the MAGE antigen may contain reduced disulphide bridges to form free thiols which have been derivatised, e.g. with carboxamide or carboxymethyl groups, see WO99/40188 In particular, MAGE antigens may suitably be employed in the form of a fusion protein with *Haemophilus influenzae* B protein D or a portion thereof or derivative thereof. For example approximately the first third of protein D or the N-terminal 100 to 110 amino acids of protein D may be employed as the fusion partner, see WO99/40188.

In a further embodiment, the antigen or antigenic composition may be a derivative of any of the antigens described herein. As used herein the term "derivative" refers to an antigen that is modified relative to its naturally occurring form. Derivatives of the present invention are sufficiently similar to native antigens to retain antigenic properties and remain capable of allowing an immune response to be raised against the native antigen. Whether or not a given derivative raises such an immune response may be measured by a suitable immunological assay such as an ELISA or flow cytometry.

The term "fragment" as used herein refers to fragments of a tumour associated antigen or derivative of the antigen which contain at least one epitope, for example a CTL epitope, typically a peptide of at least 8 amino acids. Fragments of at least 8, for example 8-10 amino acids or up to 20, 50, 60, 70, 100, 150 or 200 amino acids in length are considered to fall within the scope of the invention as long as the fragment demonstrates antigenicity, that is to say that the major epitopes (e.g. CTL epitopes) are retained by the fragment and the fragment is capable of inducing an immune response that cross-reacts with the naturally occurring tumour associated antigen. Exemplary fragments may be 8-10, 10-20, 20-50, 50-60, 60-70, 70-100, 100-150, 150-200 amino acid residues in length (inclusive of any value within these ranges).

The present invention provides an immunogenic composition as described herein for use in medicine, in particular in the treatment and/or prevention of disease. The present invention further provides an immunogenic composition as described herein for use in the immunotherapeutic treatment of cancer.

In specific examples of this embodiment the invention provides an immunogenic composition as described herein for use in the immunotherapeutic treatment of one or more cancers selected from the group consisting of prostate, breast, colorectal, lung, pancreatic, renal, ovarian or melanoma cancers.

The present invention further provides a method of therapy or prophylaxis of cancer in an individual in need thereof comprising the step of providing said individual with an effective amount of an immunogenic composition as described herein.

In specific examples of this embodiment the invention provides a method of therapy or prophylaxis of a cancer selected from the group consisting of prostate, breast, colorectal, lung, pancreatic, renal, ovarian or melanoma cancers.

The present invention will now be further described by means of the following non-limiting examples.

### Examples

### Example 1: Preparation of adjuvant composition ASA (sorbitol)

An adjuvant composition was prepared which comprised 3-deacylated MPL and QS21 in a liposomal formulation. This was prepared as follows:

### A. Method of preparation of liposomes:

A mixture of lipid (such as synthetic phosphatidylcholine), cholesterol and 3-O-deacylated MPL in organic solvent was dried down under vacuum. An aqueous solution (such as phosphate buffered saline [100mM NaCl, 20mM Phosphate pH 6.1]) was then added and the vessel agitated until all the lipid was in suspension. This suspension was then prehomogenized with high shear mixer and then high pressure homogenized until the liposomes size was reduced to around 90nm+/-10nm measured by DLS. Liposomes were then sterile filtered.

### B. ASA formulation:

### Step 1: Dilution of concentrated liposomes

Na₂/K Phosphate buffer 100mM pH6.1 when diluted 10 times was added to water for injection to reach a 10mM phosphate buffer concentration in the final formulation. A 30% (w/v) sorbitol solution in water for injection (WFI) was then added to reach a concentration of 4.7% in the final formulation - this was stirred for 15 to 45 minutes at room temperature.

Concentrated liposomes (made of DOPC, cholesterol and MPL at 40mg/ml, 10mg/ml and 2mg/ml respectively) were then added to the mix to reach a concentration of 100µg/ml of MPL in the final formulation.

The mixture was subsequently stirred for 15 to 45 minutes at room temperature.

### Step 2: QS21 addition

Using a peristaltic pump, QS21 bulk stock (thawed 24H at RT or 2 days at 4°C for 200ml) was added with a peristaltic pump at a rate of 200ml/min to the diluted liposomes under magnetic stirring to reach a 100µg/ml concentration in the final formulation. The mix was stirred for 15 to 45 minutes.

Final ASA formulation contained 100µg MPL/ml and 100µg QS21/ml.

### Step 3: pH was checked to be 6.1+/-0.3

### Step 4: Sterile filtration

Sterile filtration was realized at a constant rate of 400ml/min on a polyethersulfone (PES) filter from PALL Corporation.

### Step 5: Storage at +2°C to +8°C.

The adjuvant composition was obtained, which comprised 3-O-deacylated MPL and QS21 in a liposomal formulation and containing sorbitol (designated ASA (sorbitol)), was then stored at 4°C.

### Example 2: Preparation of adjuvant composition ASA (150mM NaCl)

### A. Method of preparation of liposomes:

A mixture of lipid (such as synthetic phosphatidylcholine), cholesterol and 3 -deacylated MPL (3D-MPL) in organic solvent was dried down under vacuum. phosphate buffered saline was then added and the vessel agitated until all the lipid is in suspension. This suspension WAs then prehomogenized with high shear mixer and then high pressure homogenized until the liposomes size was reduced to around 90nm+/-10nm measured by DLS. Liposomes were then sterile filtered on 0.22µm PES membrane.

### B. ASA formulation:

### Step 1: Dilution of concentrated liposomes

Na₂/K Phosphate buffer 100mM pH 6.45 when diluted 10 times and NaCl 1.5M were added to water for injection to reach respectively 10mM phosphate and NaCl 150mM concentrations in the final formulation. This mixture was stirred for 5 minutes at room temperature. Concentrated liposomes (made of DOPC, cholesterol and MPL at 40mg/ml, 10mg/ml and 2mg/ml respectively) were then added to the mix to reach a concentration of 100µg/ml of MPL in the final formulation. The mixture was subsequently stirred for 5 to 15 minutes at room temperature.

### Step 2: QS21 addition

QS21 bulk stock (thawed 24H at RT or 2 days at 4°C for 200ml) was added to the diluted liposomes under magnetic stirring to reach a 100µg/ml concentration in the final formulation. The mix was stirred at RT.

### Step 3: pH was checked so as to be 6.1+/-0.1.

### Step 4: Sterile filtration

Sterile filtration was realized on a polyethersulfone (PES) filter from PALL Corporation.

### Step 5: Storage at +2°C to +8°C

Final composition of ASA was 2mg DOPC, 500µg cholesterol, 100µg 3-O-deacylated MPL, 100µg QS21 per 1 ml.

### Example 3. QS21 Lytic Activity

QS21 is known to lyse red blood cells (RBC). The ASA (sorbitol) adjuvant composition prepared as in Example 1 was tested to ensure that QS21 lytic activity was quenched in the same way as was seen with the equivalent adjuvant composition comprising 150mM NaCl (ASA (150mM NaCl)).

QS21 lytic activity was measured by a haemolysis assay using chicken Red Blood cells (RBC). RBC were centrifuged at 550g at 4°C. Supernatant was discarded. The pellet was carefully resuspended in PBS buffer to reach the initial volume and the same operation was repeated until supernatant was no longer red (generally 3 times). The pellet was stored at 4°C for 3 to 4 days maximum if not used directly (and washed again the day it is used) or was diluted around 10 times in buffer if used the same day.

A QS21 dose range curve was prepared in ASA buffer (in salt or in sorbitol buffer following the ASA sample tested) extemporaneously and the adjuvant samples (containing a 50µg or 90µg equivalent of QS21 meaning the equivalent of 500µl or 900µl ASA) were prepared. Final volume was adjusted to 900µl in standards and samples with adequate buffer (containing or not sorbitol as a function of the buffer of the sample tested). Due to its opalescence, ASA interferes in optical density (OD). ASA "blanks" were thus prepared and their OD was subtracted from the OD of ASA tested samples. Those blanks corresponded to the same ASA volume as the volume tested in samples, but adjusted to 1ml with buffer. No RBC were added to these blanks. Standards and samples were then incubated with RBC (100µl of diluted RBC added to 900µl of standards and samples) for 30 minutes at room temperature (RT). Samples were then centrifuged 5 minutes at 900g. Optical density at 540nm was measured after centrifugation.

Determination of lytic activity was carried out by a limit test.
1. Limit of detection (LOD) was defined as the lowest concentration of QS21 leading to an OD: -Higher than the base level (OD>0.1)
   - Around three times higher than OD's buffer (the "o pg" QS21)
   - In the ascendant part of the curve
   - Determined for each test.
2. QS21 lytic activity was held to be positive in the adjuvant samples if the OD for the adjuvant sample was greater than the OD_{LOD}.

### Example of QS21 curve:

| ug QS21 | OD | QS21 quenched |
|---|---|---|
| 0 | 0.029 | NA |
| 0.5 | 0.052 | < LOD |
| 0.6 | 0.073 | < LOD |
| 0.7 | 0.091 | < LOD |
| 0.8 | 0.096 | < LOD |
| 0.9 | 0.12 | > 98.2% |
| 1 | 0.195 | > 98% |
| 1.1 | 0.212 | > 97.8% |
| 1.2 | 0.348 | > 97.6% |
| 1.3 | 0.479 | > 97.4% |
| 1.4 | 0.612 | > 97.2% |
| 1.5 | 0.669 | > 97% |
| 2 | 1.139 | > 96% |
| 2.5 | 1.294 | > 95% |
| 3 | 1.391 | > 94% |
| 5 | 1.416 | > 90% |
| Adjuvant * | 0.03 | > 98.2 % |

| | | |
|---|---|---|
| *50ug QS21 equivalent tested. 150mM sodium chloride buffer. | | |

The Limit of Detection in this assay is at 0.9ug QS21, and OD of 0.12

The QS21 quenching in an adjuvant composition comprising 150mM sodium chloride was estimated to be more than 98.2% for the equivalent of 50µg QS21 tested. In the case of an equivalent of 90µg tested, conclusion is more than 99%.

QS21 quenching was then compared with an equivalent adjuvant composition comprising sorbitol and only 5mM sodium chloride. Data were generated after storage of the ASA at 4°C or after accelerated stability (7days at 37°C). For the ASA in sorbitol, the QS21 standard curve was realized in a sorbitol containing buffer.

| **Sample** | **Timepoint** | **LOD** | **QS21 quenched** |
|---|---|---|---|
| Adjuvant composition (ASA) 150mM NaCl | T0 | < 1.4 | > 97.2% |
| | 7d 37°C | < 0.9 | > 98.2% |
| Adjuvant Composition (ASA) sorbitol, 5mM NaCl | T0 | < 2 | > 97.8% |
| | 7D 37°C | <1 | > 96% |
| | 11M 4°C | <2 | > 97.8%* |

| | | | |
|---|---|---|---|
| Equivalent of 50µg QS21 tested except * equivalent of 90µg QS21 tested. | | | |

It was concluded that QS21 was adequately quenched in a low sodium chloride buffer.

### Example 4 : MPL congeners.

Chemically, 3D-MPL is a mixture of 3-deacylated monophosphoryl lipid A with 4, 5 or 6 acylated chains. Each separate 3D-MPL molecule is called a congener. It is important that the congener composition remain constant, with no shift between the proportion of congeners. It is also important that any buffer used enables the congener composition to be the same as in the concentrated liposomes used to make the adjuvant compostion.

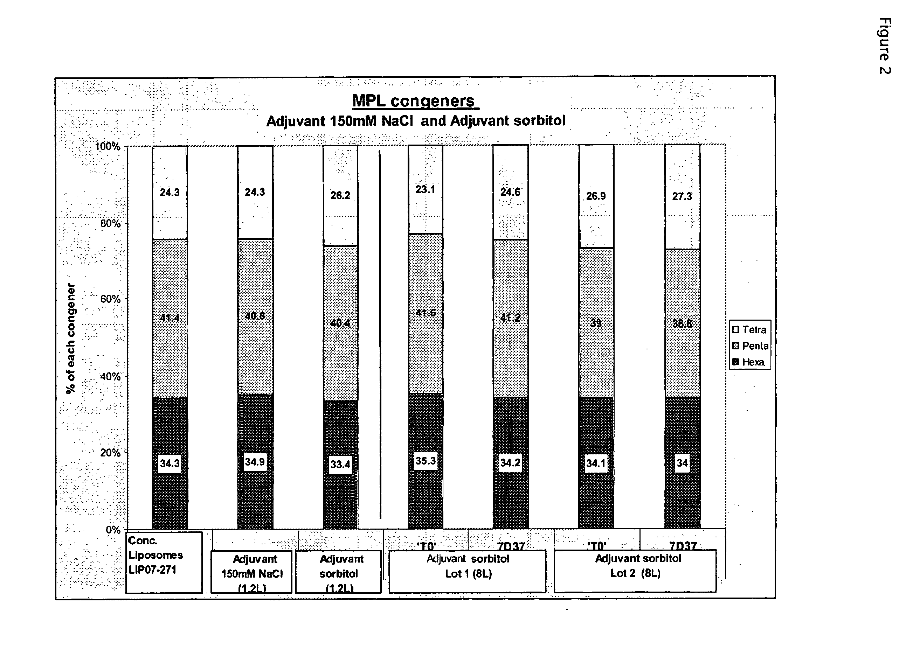

As shown on Figure 2, the congener composition was examined in 3D-MPL concentrated liposomes (Conc. Liposomes LIP07-217, first column of Figure 2), an adjuvant composition comprising 3D-MPL liposomes and QS21 in a 150mM NaCl buffer (Adjuvant 150mM NaCl, or ASA (150mM NaCl), second column), and an adjuvant composition comprising 3D-MPL liposomes and QS21 in a sorbitol and 5mM NaCl buffer (Adjuvant Sorbitol, or ASA (sorbitol), columns 3-7).

The congener composition was also examined in two lots of ASA (sorbitol) adjuvant at day 0 and 7 days after preparation and maintenance at 37 °C to ensure that there was no evolution over time (see final four columns of Figure 2).

Relative distribution of tetra-, penta- and hexa-acylated congeners of MPL in concentrated liposomes or ASA (sorbitol) samples was determined by IP-HPLC-Fluo detection (ARD). Both standards and samples were derivatised with dansylhydrazine, which introduces a Fluo-active chromophore on the dissacharide backbone. The derivatised samples were analysed on a C18 reverse phase column using tetrabutylammonium hydroxide (TBAOH) as an ion pair reagent. Congeners containing the same numbers of fatty acyl groups were eluted in distinct groups (tetraacyl, pentaacyl, and hexaacyl). Distribution of congeners is deduced by comparing the peak area of each group to the total peak area of all MPL congeners.

Figure 2 shows the percent of each congener. No significant difference in congener composition was found between adjuvant buffers, and the congener composition was consistent over time in the sorbitol buffer.

### Example 5: Preparation of compositions and used in Examples 6 and 7.

### 5.1 Preparation of PRAME with CpG (CpG 2006 is used in all Examples)

### 5.1.1 Preparation of PRAME with CpG with ASA (150mM NaCl) used in Example 6 and Example 7 experiment 1 and experiment 2

30% (w/v) sucrose solution (prepared in water for injection) was added to water for injection to reach a sucrose concentration of 5%. Tris-HCl buffer 1oomM pH 9.5 was then added to reach a 75 mM Tris buffer concentration. Borate buffer 100 mM pH 9.8 was then added to reach a 5 mM Borate buffer concentration. 10% (w/v) Poloxamer188 solution (prepared in water for injection) was then added to reach a concentration of 0.313%. The mixture was magnetically stirred (150 rpm) for 5 minutes at RT. CpG solution at concentration of about 20 mg/ml (in water for injection) was then added to reach a concentration of 1050 µg/ml in the final formulation. The mixture was magnetically stirred (150 rpm) for 5 minutes at RT. PRAME antigen was then added to reach a protein concentration of 1250 µg/ml. The mixture was magnetically stirred (150 rpm) for 15 minutes at RT. The pH was checked (9.51). The mixture obtained was filled by 0.5 ml in 3 ml glass vials then freeze dried.

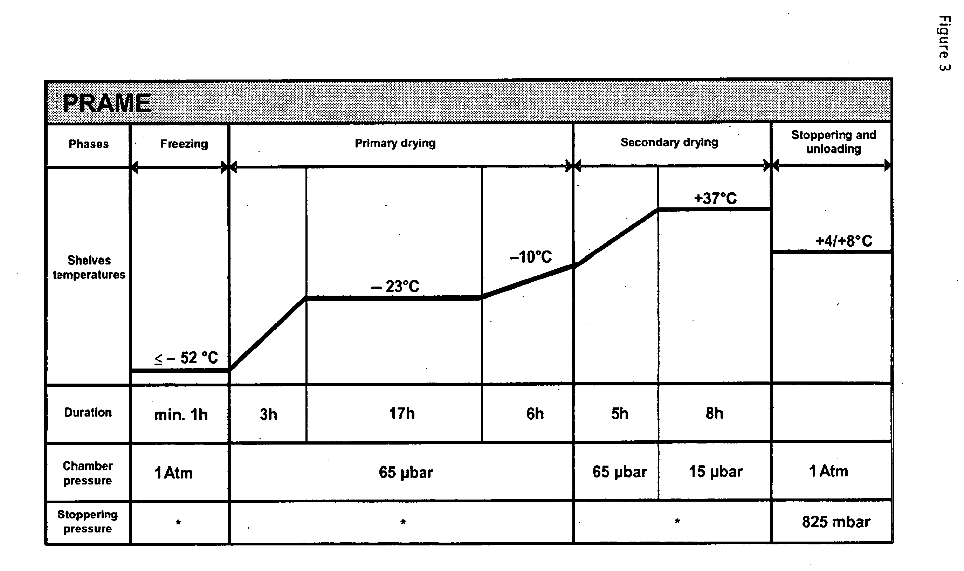

Figure 3 illustrates the freeze-drying cycle used for PRAME (duration= 40h).

The resulting lyophilsation cake was reconstituted with 625µl of aqueous adjuvant composition prepared as in Example 2 comprising 150mM NaCl. The lyophilsation cake contained a 1.25 fold excess of antigen dose to have the right antigen/adjuvant ratio after reconstitution with a final composition of 16mM Tris, 4mM borate, 4% sucrose, 0.24% Poloxamer 188, 840µg/ml CpG and 1000 µg/ml PRAME.

### 5.1.2 Preparation of PRAME with CpG for "liquid formulation" 70mM NaCl) in Experiment 1 of Example 7.

### Concentrated adjuvant preparation

PBS mod 10 fold concentrated pH 6.1 when diluted 10 times was added to water for injection to reach a 1 fold concentrated buffer in the final formulation. A premixed solution made of liposomes and QS21 prediluted at 400µg/ml was prepared separately. The premix was magnetically mixed for 15 min at room temperature. Concentrated liposomes used in the premix are made of 40mg/ml DOPC, 10mg/ml cholesterol and 2mg/ml 3-deacylated MPL. The premix was added to the PBS to reach an MPL concentration of 200µg/ml and a QS21 concentration of 200µg/ml in the final formulation. The mixture was magnetically stirred for 15 to 30 minutes at RT. CpG at around 23mg/ml was then added to reach a final concentration of 1680µg/ml. The mixture was magnetically stirred for 15 to 30 minutes at RT. pH was checked so as to be 6.1+/-0.1. The AS was filtered on 0.22µm PES filter and stored at 4°C until use.

### Final formulation

Sucrose 25%, borate 25mM pH9.8 and Lutrol 10% were added to WFI to reach respectively 9.25%, 5mM and 0.24% in the final formulation. The 2 fold concentrated AS + CpG preparation was added resulting in 1 fold concentrated in the final formulation. Mixture was stirred for 5 minutes at RT. PRAME antigen in sucrose 3.15%, borate 5mM was then added and the mixture was stirred for 5 minutes at RT.

### 5.1.3 Preparation of PRAME with CpG for "liquid formulation" in Experiment 2 of Example 7.

### Concentrated adjuvant preparation

ASA for liquid formulation was prepared as follows. Phosphate buffer 1 M (pH 6.1 when diluted 100 fold) was added under magnetic stirring to WFI to reach a 45mM final taking into account the 50mM phosphate concentration in the concentrated liposomes. Sorbitol 35% was then added to reach a 21.15% concentration final. Concentrated liposomes made of 40mg/ml DOPC, 10mg/ml cholesterol and 2mg/ml 3-deacylated MPL were added to the mixture to reach a final MPL concentration of 450µg/ml. QS21 bulk (at around 5000µg/ml) was added to reach a final QS21 concentration of 450µg/ml. The mixture is stirred for 15 minutes at RT. The pH was checked and adjusted to pH 6.1+/- 0.1. Final concentration in this ASA were respectively 450µg/ml for MPL, 450µg/ml for QS21, 45mM phosphate, 22.5 mM NaCl, 21.15% sorbitol.

### Final formulation

30% (w/v) sucrose solution (prepared in water for injection) was added to water for injection to reach a sucrose concentration of 4% in final formulation Tris-HCl buffer 1M pH 9.0 was then added to reach a 16 mM Tris buffer concentration in the final formulation. Borate buffer 100 mM pH 9.8 was then added to reach a 4 mM Borate buffer concentration in the final formulation. 10% (w/v) Poloxamer188 solution (prepared in water for injection) was then added to reach a concentration of 0.24% in the final formulation. The mixture was magnetically stirred (150 rpm) for 5 minutes at RT. CpG solution at concentration of about 20 mg/ml (in water for injection) was then added to reach a concentration of840 µg/ml in the final formulation. The mixture was magnetically stirred (150 rpm) for 5 minutes at RT. PRAME antigen buffer (Borate 5 mM - Sucrose 3.15% pH 9.8) was then added to adjust PRAME antigen concentration at 1000 µg/ml. PRAME antigen was then added to reach a protein concentration of 8 µg/ml in the final formulation. The mixture was magnetically stirred (150 rpm) for 15 minutes at RT. A 4.5 fold concentrated AS in sorbitol was added to reach final concentrations of 100µg/ml MPL and QS21. The pH was checked (+/-8.0).

### 5.1.4 Preparation of PRAME with CoG for ASA (sorbitol) and ASA (sucrose) in Experiment 1 of Example 7

30% (w/v) sucrose solution (prepared in water for injection) was added to water for injection to reach a sucrose concentration of 5% in formulation, borate buffer 100 mM pH 9.8 was then added to reach a 5 mM Borate buffer concentration in this formulation, Tris-HCl buffer 100mM pH 9.0 when 20 fold diluted was then added to reach a 5 mM Tris buffer concentration in this formulation. 10% (w/v) Poloxamer188 solution (prepared in water for injection) was then added to reach a concentration of 0.3% in the formulation. The mixture was magnetically stirred (150 rpm) for 5 minutes at RT. CpG solution at concentration of about 20 mg/ml (in water for injection) was then added to reach a concentration of 1050 µg/ml in the formulation. The mixture was magnetically stirred (150 rpm) for 5 minutes at RT. PRAME antigen was then added to reach a protein concentration of 1250 µg/ml in the final formulation. The mixture was magnetically stirred (150 rpm) for 15 minutes at RT. The pH was measured to 9.4. The mixture obtained was filled by 0.5 ml in 3 ml glass vials then freeze dried.

ASA (sorbitol) was prepared as mentioned in example 1 with some slight differences: sorbitol concentration was 4.6% and QS21 was prediluted at 400µg/ml prior to addition to the diluted concentrated liposomes.

ASA (sucrose) was prepared as mentioned in example 1 with the following differences: sorbitol is replaced by sucrose (a stock solution of 30% w/v sucrose solution is used and final sucrose concentration is 8.3%) and QS21 was prediluted at 400µg/ml prior to addition to the diluted concentrated liposomes.

The resulting lyophilisation cake was reconstituted with 625µl of aqueous adjuvant composition and the final composition comprised 4mM Tris, 4mM borate, 4% sucrose, 0.24% Poloxamer 188, 840µg/ml CpG and 1000 µg/ml PRAME.

### 5.1.5 Preparation of PRAME with CpG for ASA (sorbitol) in Experiment 2 of Example 7

30% (w/v) sucrose solution (prepared in water for injection) was added to water for injection to reach a sucrose concentration of 5% in formulation, Tris-HCl buffer 1 M pH 9.0 when 50 fold diluted was then added to reach a 20 mM Tris buffer concentration in this formulation, borate buffer 100 mM pH 9.8 when 20 fold diluted was then added to reach a 5 mM Borate buffer concentration in this formulation. 10% (w/v) Poloxamer188 solution (prepared in water for injection) was then added to reach a concentration of 0.3% in the formulation. The mixture was magnetically stirred (150 rpm) for 5 minutes at RT. CpG solution at concentration of about 20 mg/ml (in water for injection) was then added to reach a concentration of 1050 µg/ml in the formulation. The mixture was magnetically stirred (150 rpm) for 5 minutes at RT. PRAME antigen was then added to reach a protein concentration of 1250 µg/ml in the final formulation. The mixture was magnetically stirred (150 rpm) for 15 minutes at RT. The pH was measured to 9.1. The mixture obtained was filled by 0.5 ml in 3 ml glass vials then freeze dried.

ASA sorbitol was prepared as described in Example 1. The resulting lyophilisation cake was reconstituted with 625µl of aqueous adjuvant composition and the final composition comprised 16mM Tris, 4mM borate, 4% sucrose, 0.24% Poloxamer 188, 840µg/ml CpG and 1000 µg/ml PRAME.

### 5.2 Preparation of NY-ESO1 with CpG

### 5.2.1 Preparation of NY-ESO1 with CpG in Example 6.

Na/K₂ Phosphate buffer 200mM pH 6.3 when diluted 20 times was added under magnetic stirring to water for injection to reach 12.5mM in the final formulation. The following excipients were then added to the mixture and in the following order: monothioglycerol at 10% w/v to reach 0.3125% final, Poloxamer 188 at 5% w/v to reach 0.0625%, sucrose 25% to reach 5% final and L-Arginine base 287mM to reach 6.25mM. CpG bulk at around 20mg/ml was then added to this mixture to reach a concentration of 1050µg/ml in the final formulation. The mixture was maintained under magnetic stirring (around 150rpm) at room temperature for 10 minutes. The pH was checked and so as to be 7.1+/-0.3. The magnetic stirring was increased to create a vortex. NY-ESO1 was then added to reach a final concentration of 750µg/ml. The magnetic stirring was then decreased to around 150rpm and the mixture was stirred at room temperature for 5 minutes. An aliquot was taken to check the final pH (that has to be 7.02). The final bulk was then freeze- dried.

The resulting lyophilsation cake was reconstituted with 625µl of ASA buffers (150mM NaCl and sorbitol).

### Example 6. Prevention of "salting out" in the Adjuvant composition comprising sorbitol as buffer.

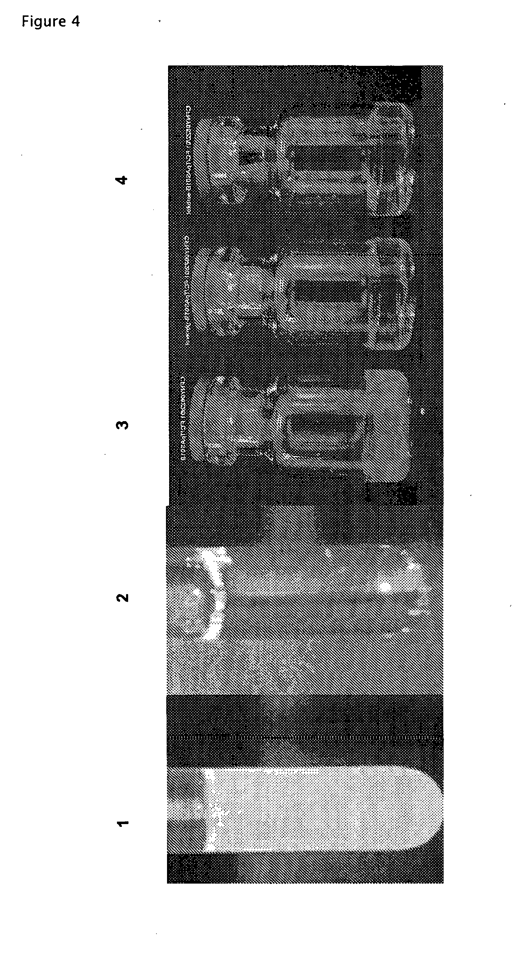

Figure 4 demonstrates that reducing the salt concentration to 5mM and including sorbitol in the adjuvant composition prevents "salting out" of both PRAME and NY-ESO-1 in immunogenic compositions (as prepared in Example 5.1.1 and 5.2.1 respectively). In Figure 4:
1. PRAME antigen reconstituted in ASA 150mM NaCl buffer.
2. PRAME antigen reconstituted in ASA sorbitol buffer.
3. NYESO-1 antigen reconstituted in ASA 150mM NaCl buffer.
4. NYESO-1 antigen reconstituted in ASA sorbitol buffer.

Figure 4 is a photographic comparison between PRAME and NYESO-1 reconstituted in ASA (150mM NaCl) and ASA (sorbitol). As can be seen, PRAME reconstituted in ASA (150mM NaCl) appears cloudy compared to PRAME reconstituted in ASA (sorbitol). Similarly, NY-ESO reconstituted in ASA (150mM NaCl) appears cloudy compared to NY-ESO antigen reconstituted in ASA (sorbitol).

### Example 7. In vivo Mouse Model

### Experiment 1:

Seven groups of twenty female CB6F1 mice (hybrid of C57BL/6 and Balb/C mice) 6 to 8 weeks old were immunized twice intra-muscularly (every two weeks, in alternative injections in left and right gastrocnemius muscle) with the PRAME protein formulated in a fixed dose of ASA +CpG which correspond to 1/10^{th} of a human dose (50µL containing 5µg MPL, 5µg QS21 and 42µg CpG7909). 14 days after the last immunization four groups of mice (n=8 each group) were challenged subcutaneously with 10e5 CT26-PRAME tumor cells, as described below. The seven groups of mice were:
- grp1: buffer
- grp2: colyophilised PRAME + CpG resuspended in the "classical" ASA (150mM NaCl) in which the PRAME protein precipitates
- grp3: colyo PRAME + CpG resuspended in ASA (sucrose)
- grp4: colyo PRAME + CpG resuspended in ASA (sucrose - incubation 48h at 25°c)
- grp5: colyo PRAME + CpG resuspended in ASA (sorbitol)
- grp6: PRAME + ASA - "liquid" formulation containing 70mM NaCl +CpG
- grp7: PRAME + ASA - "liquid" formulation containing 70mM NaCl +CpG (+ poloxamer)

7 days after the last immunization, the cellular response by the ability of T-cell to secrete cytokines (n=4 groups of 3 mice) was analyzed.

All the mice received 0.4µg of PRAME in 1/10th of Human dose of ASA +CpG Adjuvant System (5µg of MPL, 5µg of QS21 and 42µg of CpG).

### Results:

### Tumor Growth

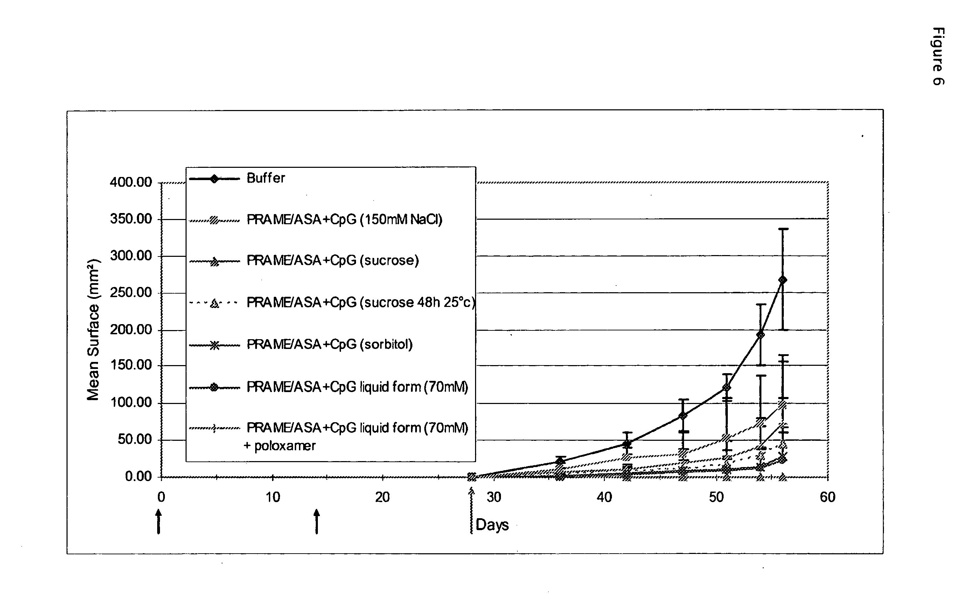

Mean tumor growth over time, as measured by surface (mm²) (+SD), per group is represented in Figure 6. The PRAME formulated ASA (150mM NaCl) +CpG and PRAME in the ASA liquid formulation induce a lower tumor protection (greater tumor growth) than the PRAME ASA (sorbitol) + CpG.

### Cellular response (7 days post 2 immunizations- n=4 groups of 3 mice per group treatment):

The frequency of CD4 and CD8 T-cells able to produce cytokines like IFNγ and TNFα after immunization of mice with the PRAME ASA-CpG tumour antigen is used to reflect the capacity of the different formulations to induce a functional cellular response. 7 days after the second immunization, the percentages of CD4 and CD8 T-cells producing cytokines (IFNγ and TNFα) were measured by intracellular cytokine staining (ICS) on spleen cells of immunized mice.

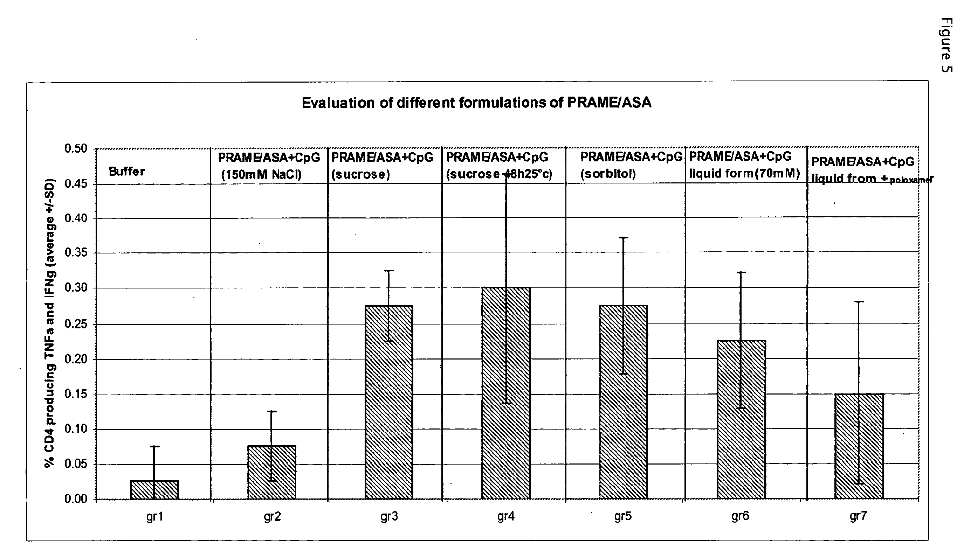

The % of CD4 producing IFNγ and TNFα (average +/- standard deviation) are shown in Figure 5.

No measurable CD8 response was found in this experiment.

The PRAME protein formulated in ASA (150mM NaCl) +CpG was shown to precipitate and induces a lower specific T-cell response compared to PRAME formulated in ASA (sorbitol) + CpG . A similarly good PRAME specific CD4 response was obtained when the PRAME protein was formulated in ASA (sorbitol) +CpG and in the ASA "liquid" formulation (70mM NaCl) + CpG (no statistical difference). The humoral immune response was also tested but the data after 2 injections were not interpretable.

### Experiment 2:

Four groups of twenty-four female CB6F1 mice (hybrid of C57BL/6 and Balb/C mice) 6 to 8 weeks old were immunized four times intra-muscularly (every two weeks, in alternative injections in left and right gastrocnemius muscle) with the PRAME protein formulated in a fixed dose of ASA +CpG which correspond to 1/10^{th} of a human dose (50µL containing 5µg MPL, 5µg QS21 and 42µg CpG7909). 14 days after the last immunization four groups of mice (n=12 each group) were challenged subcutaneously with 10e5 CT26-PRAME tumor cells, as described below. The four groups of mice were:
- grp1: buffer
- grp2: colyophilised PRAME + CpG resuspended in the "classical" ASA (150mM NaCl) in which the PRAME protein precipitates
- grp3: colyo PRAME + CpG resuspended in ASA (sorbitol)
- grp4: PRAME + ASA - "liquid" formulation +CpG (+ poloxamer)

14 days after the last immunization, the immune response was analyzed using different read outs as follows:
- Humoral response (n=12)
- Cellular response by the ability of T-cell to secrete cytokines(n=4 groups of 3 mice)
- Protective effect against a tumor challenge (n=12)

All the mice received 0.4µg of PRAME in 1/10th of Hu dose of ASA +CpG Adjuvant System (5µg of MPL, 5µg of QS21 and 42µg of CpG).

### Results:

### Tumor Growth

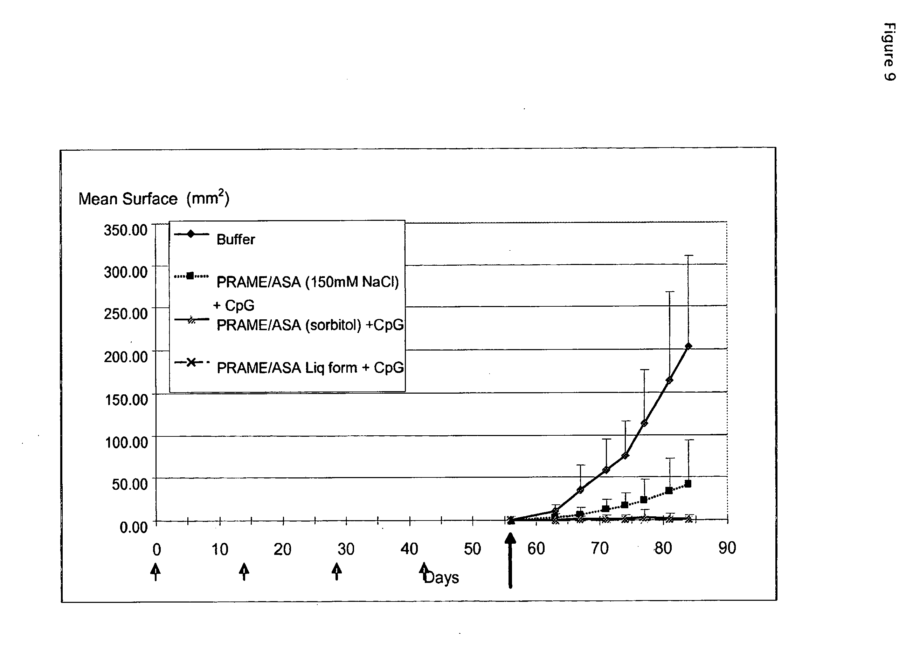

Mean tumor growth over time, as measured by surface (mm²) (+SD), per group is represented in Figure 9. The PRAME formulated ASA (150mM NaCl) +CpG induces a lower tumor protection (greater tumor growth) than the PRAME ASA (sorbitol) + CpG. Similar protection was observed for PRAME ASA (sorbitol) + CpG and PRAME in the ASA liquid formulation + CpG).

### Sample Analysis: Humoral response:

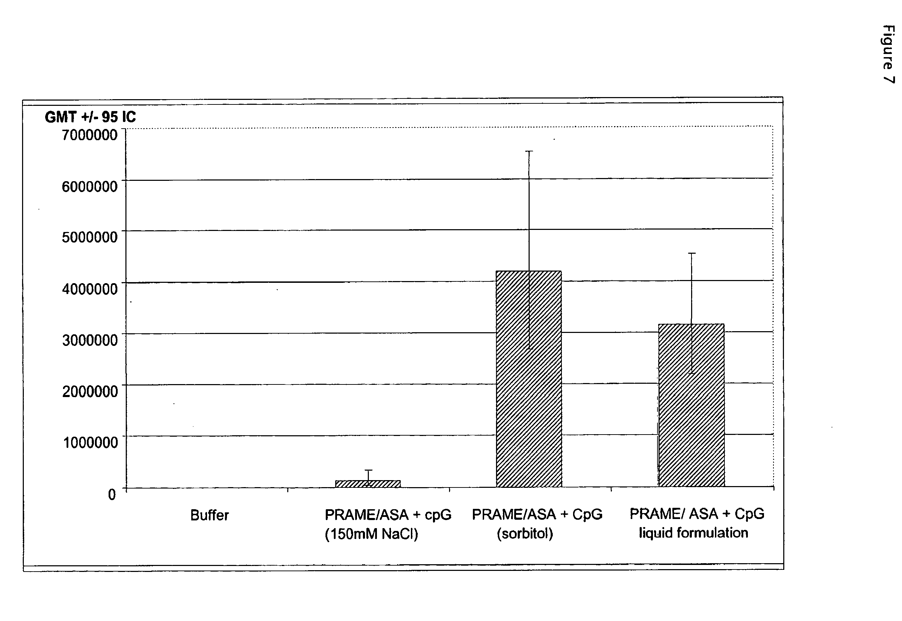

Mice sera (n=12) were tested by ELISA for the presence of PRAME-specific antibodies 14 days after the last of the 4 immunizations, as described below. The antibody response (total Ig) was assessed by ELISA using the purified recombinant PRAME protein as coating antigen. Sera from immunized animals were analyzed for the presence of PRAME specific antibodies. The geometric mean of the Standard titers (n=12 mice) +/- 95% confidence intervals obtained after the 4 immunizations are shown in the Figure 7. The PRAME/ASA +CpG containing the classical ASA (150mM NaCl) induces a very small antibody response while the PRAME/ASA +CpG containing ASA (sorbitol) induces very high antibody titers. This response is similar to that induced by the liquid formulation ASA.

### Cellular response (14 days post 4th immunizations- n=4 groups of 3 mice per group treatment):

The frequency of CD4 and CD8 T-cells able to produce cytokines like IFNγ and TNFα after immunization of mice with the PRAME ASA-CpG tumour antigen is used to reflect the capacity of the different formulations to induce a functional cellular response. 14 days after the fourth immunization, the percentages of CD4 and CD8 T-cells producing cytokines (IFNγ and TNFα) were measured by intracellular cytokine staining (ICS) on spleen cells of immunized mice.

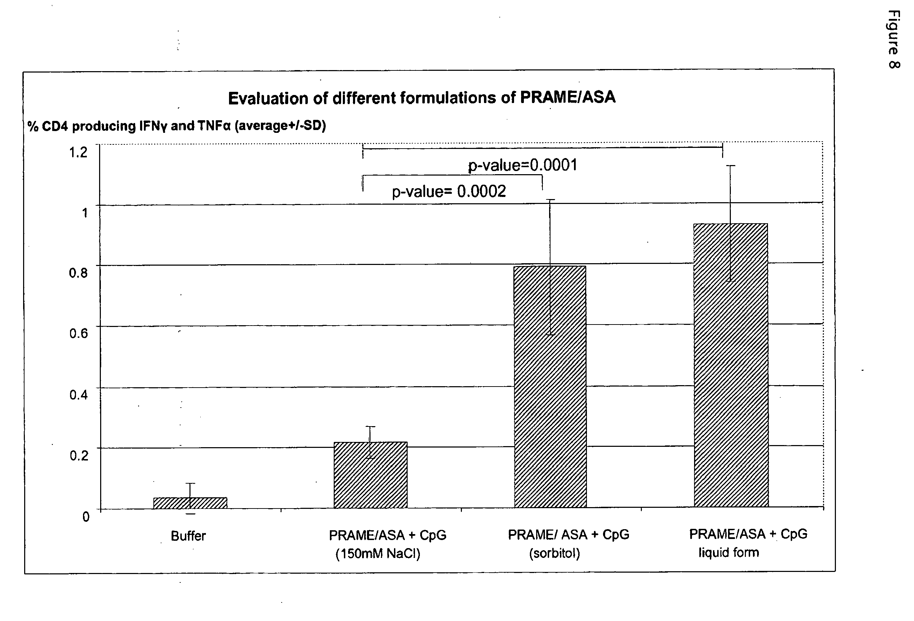

The % of CD4 producing IFNγ and TNFα (average +/- standard deviation) are shown in Figure 8. p-values are largely inferior to 0.05 demonstrating a significant difference between the group 2 and the groups 3 and 4.

No measurable CD8 response was found in this experiment.

The PRAME protein formulated in ASA (150mM NaCl) +CpG was shown to precipitate and induces a statistically lower specific T-cell response compared to PRAME formulated in ASA (sorbitol) + CpG . In contrast a similarly good PRAME specific CD4 response was obtained when the PRAME protein was formulated in ASA (sorbitol) +CpG, and in the ASA "liquid" formulation + CpG.

### Method

### CT26-PRAME tumor model and tumor growth

The CT26-PRAME cell line was generated by transfecting the CT26 colon carcinoma cell line with the mammalian expression plasmid, pCDNA3, encoding the cDNA for PRAME (Invitrogen, Carlsbad, CA). Selection with G418 (200 µg/ml) and limit-dilution cloning yielded a clone expressing PRAME (CT26-PRAME) as determined by quantitative real-time PCR (10e-3 PRAME mRNA copies / copy of mouse βactin which is in the range of the level of PRAME expression by human tumors)

CT26 PRAME cells were grown *in vitro* at 37°C with 5% CO₂ in RPMI Medium with 10% fetal calf serum, 1% L-glutamine, 1% penicillin-steptomycin, 1% non-essential amino acids, 1% sodium pyruvate and 0.1% β-mercaptoethanol. Cells were trypsinised, washed twice in serum-free medium and injected in 200µl RPMI Medium subcutaneously in the right flank of four groups of CB6F1 mice 14 days after the last immunization with PRAME as described above. Individual tumor growth was measured twice a week. The product of the 2 main diameters of each tumor was recorded overtime and the data are shown as the mean tumor surface (mm²) in each group of animals.

### Sample Analysis: Humoral response

Before addition of sera the immunoplate was coated with the PRAME antigen overnight at 4°C. After reaction with the sera for 90 mins at 37°C, a biotinylated rabbit whole antibody against mouse immunoglobulins was added for 90 mins at 37°C. The antigen-antibody complex was revealed by incubation with a streptavidin-biotinylated peroxydase complex for 30 mins at 37°C. This complex was then revealed by the addition of tetramethyl benzidine (TMB) for 10 mins at Room Temperature and the reaction was stopped with 0.2 M H₂SO₄. Optical densities were recorded at 450 nm.

### Sample Analysis Method: Cellular response (IFNg/TNFa production)

The IFNg and TNFa production by CD4 and CD8 T-cells was measured by flow cytometry (LSR2 from Becton Dickinson) using intracellular cytokine staining (ICS) on spleen cells of immunized mice ( 4 groups of 3 mice per group) after 2hrs stimulation with a pool of overlapping 15mer peptides covering the entire PRAME protein sequence.

### Stimulation of T cells:

- Spleen cells of immunized animals were re-stimulated with a pool of 123 15mer peptides overlapping by 11 amino acids, covering the entire sequence of PRAME. The peptides (1µg/ml/peptide) are mixed with 10e6 T cells (spleen cells) 2hrs at 37°c in a 96 well plate (U) in a final volume of 200µl of RPMI 5% FCS containing 2µg/ml of anti CD49d and anti CD28 at 2µg/ml
- after the incubation, 50µl of brefeldin (1/1000) was added in RPMI 5% FCS

### Intracellular staining:

- CD4/CD8 staining:
   - Cells were transferred in a 96 well plate (conic wells)
   - Centrifugation 1000rpm 5' at 4°c
   - Wash with 250µl FACS buffer (PBS 1% FCS)
   - Pellets of cells were incubated with 50µl of 2.4G2 1/50 in FACS buffer during 10' at 4°c
   - 50µl of Master mix CD4-PE (dilution Mab: 1/200) and CD8PerCP (dilution Mab: 1/200) in FACS buffer were added during 30' at 4°c
   - Wash in FACS buffer (1200RPM-10')
- Permeabilisation of cells:
   - Pellets were incubated with 200µl of cytoFix-cytoPerm solution during 20' at 4°c
   - Washed with permWASH 1x (1200RPM-10')- the permWASH solution is 10x concentrated, dilution in sterile water
- I IFNγ and TNFα intracellular staining:
   - pellets were incubated 2hrs at 4°c with 50µl of Mix IFNγ APC (dilution Mab: 1/50) and TNFα FITC (dilution Mab: 1/50) in the permWASH1x solution
   - Washed with permWASH 1X (1200RPM-10')
   - pellets were resuspended in FACS buffer
   - FACS analysis

Brefeldin (Gologi Plug): BD cat.555029
Cytofix / cytoperm: Pharmingen (BD) Cat n° 554722
Perm/wash buffer: Pharmingen(BD) Cat n° 554723
Rat anti Mouse CD49d purified NA LE : BD Cat n° 553154
Rat anti Mouse CD28 purified NA LE: BD Cat n° 553295
Rat anti Mouse CD8a perCp: BD Cat n° 553036
Rat anti Mouse CD4 PE: BD Cat n° 556616
Rat anti Mouse IFNγ APC: BD Cat n° 554413
Rat anti Mouse TNFα FITC: BD Cat n°554418
Anti- mouse CD16/CD32 (2.4G2) Becton Dickinson cat n° 553142 (0.5mg/ml)

## Claims

1. An aqueous adjuvant composition comprising: (a) a TLR-4 agonist and a saponin in a liposomal formulation; and (b) a non-ionic isotonicity agent, wherein the concentration of sodium chloride or the ionic strength in the adjuvant composition is less than 100mM.

2. An aqueous adjuvant composition according to any preceding claim wherein the non-ionic isotonicity agent is a polyol.

3. An aqueous adjuvant composition according to claim 2 wherein the polyol is sorbitol.

4. An aqueous adjuvant composition of claim 3 wherein the concentration of sorbitol is between about 4% and about 10% (w/v).

5. The aqueous adjuvant composition of any preceding claim wherein said TLR-4 agonist is 3D-MPL.

6. An aqueous adjuvant composition according to any preceding wherein said saponin is QuilA or a derivative thereof.

7. An aqueous adjuvant composition according to claim 6 wherein the derivative of QuilA is QS21.

8. An aqueous adjuvant composition according to any preceding claim comprising about 5mM sodium chloride and between 5% and 6% w/v sorbitol.

9. An immunogenic composition comprising an antigen or antigenic preparation and an aqueous adjuvant composition of any one of claims 1 to 8.

10. An immunogenic composition according to claim 9 wherein said antigen or antigenic preparation is not soluble in salt concentrations or in solutions wherein the ionic strength is higher than 5mM, 10mM, 20mM, 30mM, 40mM, 50mM, 60mM, 70mM, 80mM, 90mM or 100mM.

11. A process for preparing an immunogenic composition of either claim 9 or 10 comprising the step of reconstituting a lyophilised composition comprising at least one antigen or antigenic preparation with an aqueous adjuvant composition as defined in any of claims 1 to 8.

12. A kit comprising (i) a lyophilised composition comprising an antigen or antigenic preparation and (ii) an aqueous adjuvant composition of any one of claims 1 to 8.

13. The process or kit of claims 11 or 12 wherein the antigen or antigenic preparation is not soluble in salt concentrations or in solutions wherein the ionic strength is greater than 10mM, 20mM, 30mM, 40mM, 50mM, 60mM, 70mM, 80mM, 90mM or 100mM.

14. An immunogenic composition according to claims 9 or 10 for use in medicine.

## Patentansprüche

1. Wässrige Adjuvanzzusammensetzung, umfassend: (a) einen TLR-4-Agonisten und ein Saponin in einer Liposomenformulierung; sowie (b) ein nicht-ionisches Isotonizitätsmittel, wobei die Konzentration von Natriumchlorid oder die Ionenstärke in der Adjuvanzzusammensetzung weniger als 100 mM beträgt.

2. Wässrige Adjuvanzzusammensetzung gemäß einem vorhergehenden Anspruch, wobei das nicht-ionische Isotonizitätsmittel ein Polyol ist.

3. Wässrige Adjuvanzzusammensetzung gemäß Anspruch 2, wobei das Polyol Sorbitol ist.

4. Wässrige Adjuvanzzusammensetzung gemäß Anspruch 3, wobei die Konzentration von Sorbitol im Bereich von ungefähr 4 % bis ungefähr 10 % (Gew./Vol.) liegt.

5. Wässrige Adjuvanzzusammensetzung gemäß einem vorhergehenden Anspruch, wobei der TLR-4-Agonist 3D-MPL ist.

6. Wässrige Adjuvanzzusammensetzung gemäß einem vorhergehenden Anspruch, wobei das Saponin QuilA oder ein Derivat davon ist.

7. Wässrige Adjuvanzzusammensetzung gemäß Anspruch 6, wobei das Derivat von QuilA QS21 ist.

8. Wässrige Adjuvanzzusammensetzung gemäß einem vorhergehenden Anspruch, umfassend ungefähr 5 mM Natriumchlorid und zwischen 5 % und 6 % (Gew./Vol.) Sorbitol.

9. Immunogene Zusammensetzung, umfassend ein Antigen oder eine antigene Zubereitung und eine wässrige Adjuvanzzusammensetzung gemäß einem der Ansprüche 1 bis 8.

10. Immunogene Zusammensetzung gemäß Anspruch 9, wobei das Antigen oder die antigene Zubereitung nicht in Salzkonzentrationen oder in Lösungen mit einer Ionenstärke von mehr als 5 mM, 10 mM, 20 mM, 30 mM, 40 mM, 50 mM, 60 mM, 70 mM, 80 mM, 90 mM oder 100 mM löslich ist.

11. Verfahren zum Herstellen einer immunogenen Zusammensetzung gemäß Anspruch 9 oder 10, umfassend den Schritt des Rekonstituierens einer lyophilisierten Zusammensetzung, die mindestens ein Antigen oder eine antigene Zubereitung umfasst, mit einer wässrigen Adjuvanzzusammensetzung, wie in einem der Ansprüche 1 bis 8 definiert.

12. Kit, umfassend (i) eine lyophilisierte Zusammensetzung, umfassend ein Antigen oder eine antigene Zubereitung und (ii) eine wässrige Adjuvanzzusammensetzung gemäß einem der Ansprüche 1 bis 8.

13. Verfahren oder Kit gemäß den Ansprüchen 11 oder 12, wobei das Antigen oder die antigene Zubereitung nicht in Salzkonzentrationen oder in Lösungen mit einer Ionenstärke von mehr als 10 mM, 20 mM, 30 mM, 40 mM, 50 mM, 60 mM, 70 mM, 80 mM, 90 mM oder 100 mM löslich ist.

14. Immunogene Zusammensetzung gemäß einem der Ansprüche 9 oder 10 für die medizinische Verwendung.

## Revendications

1. Composition aqueuse d'adjuvant comprenant : (a) un agoniste de TLR-4 et une saponine dans une formulation liposomale ; et (b) un agent d'isotonicité non ionique, dans laquelle la concentration de chlorure de sodium ou la force ionique dans la composition d'adjuvant est inférieure à 100 mM.

2. Composition aqueuse d'adjuvant selon la revendication précédente, dans laquelle l'agent d'isotonicité non ionique est un polyol.

3. Composition aqueuse d'adjuvant selon la revendication 2, dans laquelle le polyol est le sorbitol.

4. Composition aqueuse d'adjuvant selon la revendication 3, dans laquelle la concentration en sorbitol est entre environ 4 % et environ 10 % (p/v).

5. Composition aqueuse d'adjuvant selon l'une quelconque des revendications précédentes, dans laquelle ledit agoniste de TLR-4 est le 3D-MPL.

6. Composition aqueuse d'adjuvant selon l'une quelconque des revendications précédentes, dans laquelle ladite saponine est QuilA ou un dérivé de celle-ci.

7. Composition aqueuse d'adjuvant selon la revendication 6, dans laquelle le dérivé de QuilA est QS21.

8. Composition aqueuse d'adjuvant selon l'une quelconque des revendications précédentes, comprenant environ 5 mM de chlorure de sodium et entre 5 % et 6 % p/v de sorbitol.

9. Composition immunogène comprenant un antigène ou une préparation antigénique et une composition aqueuse d'adjuvant selon l'une quelconque des revendications 1 à 8.

10. Composition immunogène selon la revendication 9, dans laquelle ledit antigène ou ladite préparation antigénique n'est pas soluble dans des concentrations en sel ou dans des solutions dans lesquelles la force ionique est supérieure à 5 mM, 10 mM, 20 mM, 30 mM, 40 mM, 50 mM, 60 mM, 70 mM, 80 mM, 90 mM ou 100 mM.

11. Procédé de préparation d'une composition immunogène selon la revendication 9 ou 10, comprenant l'étape de reconstitution d'une composition lyophilisée comprenant au moins un antigène ou une préparation antigénique avec une composition aqueuse d'adjuvant telle que définie dans l'une quelconque des revendications 1 à 8.

12. Nécessaire comprenant (i) une composition lyophilisée comprenant un antigène ou une préparation antigénique et (ii) une composition aqueuse d'adjuvant selon l'une quelconque des revendications 1 à 8.

13. Procédé ou nécessaire selon les revendications 11 ou 12, dans lequel l'antigène ou la préparation antigénique n'est pas soluble dans des concentrations en sel ou dans des solutions dans lesquelles la force ionique est supérieure à 10 mM, 20 mM, 30 mM, 40 mM, 50 mM, 60 mM, 70 mM, 80 mM, 90 mM ou 100 mM.

14. Composition immunogène selon les revendications 9 ou 10, pour une utilisation en médecine.


### SEQUENCE LISTING

<110> Lemoine, Dominique Henderickx, Veronique
<120> Novel Compositions
<130> VB63569
<160> 5
<170> FastSEQ for Windows Version 4.0
<210> 1
   <211> 20
   <212> DNA
   <213> Artificial Sequence
<220>
   <223> Oligonucleotide
<400> 1
   tccatgacgt tcctgacgtt 20
<210> 2
   <211> 18
   <212> DNA
   <213> Artificial Sequence
<220>
   <223> Oligonucleotide
<400> 2
   tctcccagcg tgcgccat 18
<210> 3
   <211> 30
   <212> DNA
   <213> Artificial Sequence
<220>
   <223> Oligonucleotide
<400> 3
   accgatgacg tcgccggtga cggcaccacg 30
<210> 4
   <211> 24
   <212> DNA
   <213> Artificial Sequence
<220>
   <223> Oligonucleotide
<400> 4
   tcgtcgtttt gtcgttttgt cgtt 24
<210> 5
   <211> 20
   <212> DNA
   <213> Artificial Sequence
<220>
   <223> Oligonucleotide
<400> 5
   tccatgacgt tcctgatgct 20